# EUROPEAN PATENT SPECIFICATION

(11) **EP 3 501 596 B1**
(45) Date of publication and mention of the grant of the patent: **05.05.2021**
(21) Application number: 18213123.5
(22) Date of filing: 17.12.2018
(51) Int. Cl.: A61N 1/39, A61B 5/053, A61N 1/08, A61N 1/04, A61B 5/318, A61B 5/333, A61B 5/349

(54) **DETERMINATION OF CARDIOPULMONARY RESUSCITATION COMPRESSION RATE**
BESTIMMUNG DER KARDIOPULMONALEN REANIMATIONSKOMPRESSIONSRATE
DÉTERMINATION DE TAUX DE COMPRESSION DE RÉANIMATION CARDIO-PULMONAIRE

(30) Priority: 19.12.2017 GB 201721255
(43) Date of publication of application: 26.06.2019
(73) Proprietor: HeartSine Technologies Limited, Belfast Antrim BT3 9ED (GB)
(72) Inventor: McCanny, Paul, Belfast, BT3 9ED (GB); Todd, Ian, Belfast, BT3 9ED (GB)
(74) Representative: Armstrong, Rosemary

(56) References cited:
- WO-A1-2015/200813
- US-A1- 2014 100 497
- US-A1- 2016 331 330

## Description

The invention relates to the determination of cardiopulmonary resuscitation compression rate, particularly in real-time using a defibrillator.

When using a defibrillator on a subject, the defibrillator will often advise the user to perform cardiopulmonary resuscitation (CPR) on the subject. CPR is performed to ensure that oxygenated blood remains circulating in the body of the subject in the absence of a heartbeat. CPR may be advised, for example, between application of one or more defibrillation shocks to the subject or when the subject's electrocardiogram (ECG) indicates that CPR, rather than defibrillation shocks, is the best treatment. Performance of effective CPR has been shown to increase a subject's chance of survival. To be effective, CPR compressions must be performed at a rate suitable for the subject and rate determination of the CPR compressions is therefore important.

CPR compressions involve the compression and release of the chest of the subject. Commonly, accelerometers are employed with defibrillators to directly measure the motion of the chest for CPR compression rate determination. However, there are problems associated with this rate determination method. As accelerometers measure chest motion by double integration of the measured acceleration of the chest, compression rate determination from the measured chest acceleration can comprise large uncertainties and may not be accurate. In a static environment this problem may be limited, but in a real-world environment there may be motion of the subject which could lead to issues with rate determination. Accelerometers may also be subject to noisy signal inputs if they move relative to the body of the subject, further complicating CPR compression rate determination. In addition, the use of an additional sensor with the defibrillator increases the complexity of the defibrillator.

Pressure sensitive pads may be employed with defibrillators to measure the compressions applied to the subject's chest. These are also subject to issues with noise and the complication, cost and size of additional sensors.

Chest compressions performed during CPR produce changes to transthoracic impedance of the subject. The subject's chest acts as a resistor to an applied electrical current and when the volume of the chest changes due to CPR compressions, the transthoracic impedance will change. Transthoracic impedance variations may therefore be used to determine the rate of compressions. This rate determination method also has associated problems. The morphology of the impedance signal may change over time. This may be due to the subject's chest shape physically changing as a result of compressions, or perhaps because a different person is administering CPR, or the person administering CPR is fatigued. The reality of rescue of a subject in the field means moving subjects and compressions that vary in rate, force and application angle in a short space of time. The impedance signal morphology may change in such a way as to confuse conventional compression rate determination methods.

US2014/100497 discloses a method of controlling a defibrillator with a function of analyzing an electrocardiogram, including: dividing an electrocardiogram of a patient into a plurality of analysis zones; executing analysis of the electrocardiogram in each of the divided analysis zones; based on a result of the analysis of the electrocardiogram in each of the analysis zones, executing determination of whether electric shock on the patient is necessary or not, and calculating reliability of the determination; and based on a combination of the determination and the reliability, instructing a first procedure to be performed on the patient.

According to a first aspect of the invention there is provided a defibrillator for determining a rate of cardiopulmonary resuscitation compressions on a subject, comprising
electrodes adapted to be attached to the subject,
an impedance signal measurement system connected to the electrodes and configured to measure at least one impedance signal of the subject,
an electrocardiogram signal measurement system connected to the electrodes and configured to measure at least one electrocardiogram signal of the subject,
an impedance signal processing system connected to the impedance signal measurement system and configured to process the impedance signal of the subject to obtain an impedance signal frequency spectrum and to identify a plurality of peaks in the impedance signal frequency spectrum and determine central frequencies of the plurality of peaks as a plurality of impedance signal compression rate estimates and determine central frequencies and amplitudes of the plurality of peaks as a plurality of impedance signal features,
an electrocardiogram signal processing system connected to the electrocardiogram signal measurement system and configured to process the electrocardiogram signal of the subject to obtain an electrocardiogram signal frequency spectrum and to identify a plurality of peaks in the electrocardiogram signal frequency spectrum and determine central frequencies and amplitudes of the plurality of peaks as a plurality of electrocardiogram signal features,
a compression rate estimate processing system connected to the impedance signal measurement system and the electrocardiogram signal measurement system and configured to apply to the impedance signal features and the electrocardiogram signal features a plurality of criteria comprising
in the impedance signal frequency spectrum a ratio of a secondary peak amplitude and a primary peak amplitude being greater than a pre-determined impedance signal amplitude ratio threshold,
in the impedance signal frequency spectrum a central frequency of a primary peak being greater than a central frequency of a secondary peak,
in the ECG signal frequency spectrum a central frequency of a primary peak being less than a central frequency of a secondary peak,
a frequency difference between a lower frequency primary or secondary peak in the impedance signal frequency spectrum and a lower frequency primary or secondary peak in the ECG signal frequency spectrum being less than a pre-determined frequency difference threshold,
in the ECG signal frequency spectrum a ratio of a secondary peak amplitude and a primary peak amplitude being greater than a pre-determined ECG signal amplitude ratio threshold, and
use compliance with each of the criteria to select one of the plurality of impedance signal compression rate estimates comprising a central frequency of a secondary peak of the impedance signal frequency spectrum as the cardiopulmonary resuscitation compression rate, and an output unit connected to the compression rate estimate processing system and configured to output feedback based on the cardiopulmonary resuscitation compression rate to a user of the defibrillator.

The impedance signal measurement system may be configured to measure the at least one impedance signal by continuously sampling impedance data received from the electrodes. The ECG signal measurement system may be configured to measure the at least one ECG signal by continuously sampling ECG data received from the electrodes.

Processing the impedance signal of the subject to obtain the plurality of impedance signal compression rate estimates may comprise using a frequency domain transformation on the impedance signal to obtain the impedance signal frequency spectrum, using a peak detection algorithm to identify the plurality of peaks in the impedance signal frequency spectrum and determine central frequencies of the plurality of peaks as the plurality of impedance signal compression rate estimates.

Processing the impedance signal of the subject to obtain the plurality of impedance signal features may comprise using a frequency domain transformation on the impedance signal to obtain the impedance signal frequency spectrum, using a peak detection algorithm to identify the plurality of peaks in the impedance signal frequency spectrum and determine central frequencies and amplitudes of the plurality of peaks as the plurality of impedance signal features.

Processing the ECG signal of the subject to obtain the plurality of ECG signal features may comprise using a frequency domain transformation on the ECG signal to obtain the ECG signal frequency spectrum, using a peak detection algorithm to identify the plurality of peaks in the ECG signal frequency spectrum and determine central frequencies and amplitudes of the plurality of peaks as the plurality of ECG signal features.

Using the frequency domain transformation on the impedance signal to obtain the impedance signal frequency spectrum may comprise using a Fast Fourier Transform (FFT) algorithm on a window of the impedance signal. Using the frequency domain transformation on the impedance signal to obtain the impedance signal frequency spectrum may comprise using a Goertzel algorithm on a window of the impedance signal. It will be appreciated that other frequency domain transformation methods may be used.

Using the frequency domain transformation on the ECG signal to obtain the ECG signal frequency spectrum may comprise using a FFT algorithm on a window of the ECG signal corresponding to the window of the impedance signal. Using the frequency domain transformation on the ECG signal to obtain the ECG signal frequency spectrum may comprise using a Goertzel algorithm on a window of the ECG signal corresponding to the window of the impedance signal. It will be appreciated that other frequency domain transformation methods may be used.

The window of the impedance signal and the ECG signal may comprise a window size in the range of 3 seconds to 20 seconds. The window may comprise a six second window size. The window may be an advancing window. The window may advance by a period which is less than the window size. The window may advance by a period of 1 second.

Using the peak detection algorithm to identify a plurality of peaks in the impedance signal frequency spectrum may comprise identifying peaks based on decreasing steepness of slopes of the impedance signal frequency spectrum. Using the peak detection algorithm to identify a plurality of peaks in the ECG signal frequency spectrum may comprise identifying peaks based on decreasing steepness of slopes of the ECG signal frequency spectrum. It will be appreciated that some other methods of peak detection may be used. The peak detection algorithm may return a primary peak having a highest amplitude at its central frequency, a secondary peak having a next highest amplitude at its central frequency continued up to a specified number of peaks. The peak detection algorithm may use an amplitude threshold to determine if a peak is a true peak. The amplitude threshold may comprise a pre-determined number of standard deviations above background.

The impedance signal processing system may process the impedance signal to obtain a plurality of additional impedance signal features from the impedance signal comprising any of variance of the impedance signal, morphology of the impedance signal, gradient of the impedance signal, power of the impedance signal, wavelet decomposition of the impedance signal, noise analysis of the impedance signal, cepstrum of the impedance signal.

The ECG signal processing system may process the ECG signal to obtain a plurality of additional ECG signal features from the ECG signal comprising any of variance of the ECG signal, morphology of the ECG signal, gradient of the ECG signal, power of the ECG signal, wavelet decomposition of the ECG signal, noise analysis of the ECG signal, cepstrum of the ECG signal.

The impedance amplitude ratio threshold may be 0.2.

The frequency difference threshold may be 0.25 Hz. This criterion investigates whether an ECG signal frequency spectrum peak and an impedance signal frequency spectrum peak are aligned within a pre-determined limit.

The compression rate estimate processing system uses compliance with each of the plurality of criteria to select one of the plurality of impedance signal compression rate estimates as the CPR compression rate. The compression rate estimate processing system uses compliance with each of the plurality of criteria to select an impedance signal compression rate estimate comprising a central frequency of a secondary peak of the impedance signal frequency spectrum as the CPR compression rate. The compression rate estimate processing system may use non-compliance with any of the plurality of criteria to select an impedance signal compression rate estimate comprising a central frequency of a primary peak of the impedance signal frequency spectrum as the CPR compression rate.

The ECG signal amplitude ratio threshold may be 0.2.

The compression rate estimate processing system uses compliance with each of the plurality of criteria to select an impedance signal compression rate estimate comprising a central frequency of a secondary peak of the impedance signal frequency spectrum as the CPR compression rate. The compression rate estimate processing system may use non-compliance with any of the plurality of criteria to select an impedance signal compression rate estimate comprising a central frequency of a primary peak of the impedance signal frequency spectrum as the CPR compression rate.

The defibrillator may determine a CPR compression rate using substantially all of the measured impedance signal and substantially all of the measured ECG signal. The defibrillator may determine a CPR compression rate using one or more portions of the measured impedance signal and corresponding one or more portions of the measured ECG signal. The or each portion of the measured impedance signal may comprise portions which have been pre-analysed for CPR compression rate, which compression rate has not exceeded a threshold used to determine if the rate is a true rate. Thus CPR compression rate may be determined for one or more portions of the impedance signal using traditional impedance signal analysis techniques alone, and CPR compression rate may be determined for one or more portions of the impedance signal using the method of the invention which combines using the impedance signal with using the ECG signal for qualification of the CPR compression rate.

The defibrillator may use processing of the impedance signal and the ECG signal to determine features based on a morphology of the impedance signal. The defibrillator may use processing of the impedance signal and the ECG signal to determine motion of the subject.

The output unit may output feedback based on the CPR compression rate to the user of the defibrillator comprising an indication that the CPR compression rate is any of satisfactory, too slow, too fast. Outputting the feedback based on the CPR compression rate to the user of the defibrillator provides the user with an opportunity to improve the quality of CPR and gives an indication of the effectiveness of the compressions.

According to a second aspect of the invention there is provided a method of determining a cardiopulmonary resuscitation (CPR) compression rate, comprising
receiving an impedance signal of a subject,
receiving an electrocardiogram (ECG) signal of the subject,
processing the impedance signal to obtain an impedance signal frequency spectrum and to identify a plurality of peaks in the impedance signal frequency spectrum and determine central frequencies of the plurality of peaks as a plurality of impedance signal compression rate estimates,
processing the impedance signal to obtain the impedance signal frequency spectrum and to identify the plurality of peaks in the impedance signal frequency spectrum and determine central frequencies and amplitudes of the plurality of peaks as a plurality of impedance signal features,
processing the ECG signal to obtain an electrocardiogram signal frequency spectrum and to identify a plurality of peaks in the electrocardiogram signal frequency spectrum and determine central frequencies and amplitudes of the plurality of peaks as a plurality of ECG signal features, and
applying to the impedance signal features and the ECG signal features a plurality of criteria comprising
in the impedance signal frequency spectrum a ratio of a secondary peak amplitude and a primary peak amplitude being greater than a pre-determined impedance signal amplitude ratio threshold,
in the impedance signal frequency spectrum a central frequency of a primary peak being greater than a central frequency of a secondary peak,
in the ECG signal frequency spectrum a central frequency of a primary peak being less than a central frequency of a secondary peak,
a frequency difference between a lower frequency primary or secondary peak in the impedance signal frequency spectrum and a lower frequency primary or secondary peak in the ECG signal frequency spectrum being less than a pre-determined frequency difference threshold,
in the ECG signal frequency spectrum a ratio of a secondary peak amplitude and a primary peak amplitude being greater than a pre-determined ECG signal amplitude ratio threshold, and
using compliance with each of the criteria to select one of the plurality of impedance signal compression rate estimates comprising a central frequency of a secondary peak of the impedance signal frequency spectrum as the CPR compression rate.

According to a third aspect of the invention there is provided a CPR compression rate determination computer program, tangibly embodied on a computer readable medium, the computer program including instructions for causing a computer to execute the method of determining a rate of CPR compressions according to the second aspect of the invention.

The aspects of the invention allow a more robust determination of CPR compression rate than was previously possible using the impedance alone, without the need for additional sensors and using signals that are already measured in defibrillators.

Embodiments of the present invention will now be described, by way of example only, with reference to the accompanying drawings, in which:
Figure 1 is a schematic representation of a defibrillator for determining a rate of CPR compressions on a subject according to the first aspect of the invention;
Figure 2 is a flow chart representation of a method of determining a CPR compression rate carried out by the defibrillator of Figure1, and
Figure 3 is flow chart representation showing further details of the method of Figure 2.

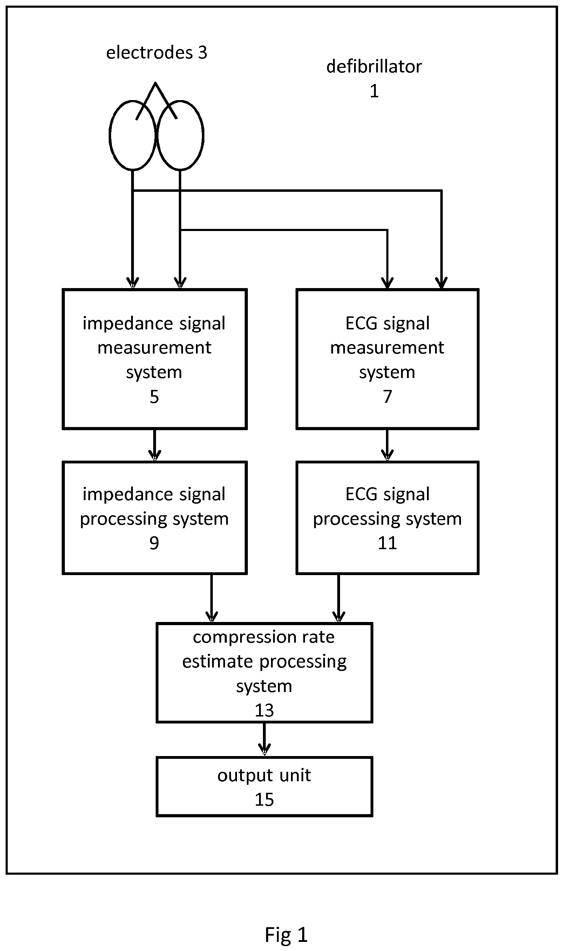

Referring to Figure 1, a defibrillator 1 for determining a rate of CPR compressions on a subject is shown. This comprises electrodes 32 adapted to be attached to the subject (not shown), an impedance signal measurement system 5 connected to the electrodes 3 and an ECG signal measurement system 7 connected to the electrodes 3. The defibrillator 1 further comprises an impedance signal processing system 9 connected to the impedance signal measurement system 5, an ECG signal processing system 11 connected to the ECG signal measurement system 7, a compression rate estimate processing system 13 connected to the impedance signal processing system 9 and the ECG signal processing system 11 and an output unit 15 connected to the compression rate estimate processing system 13.

It will be appreciated that the defibrillator 1 will comprise other elements such as defibrillation shock generation circuitry, a power source.

The impedance signal measurement system 5 comprises an impedance measurement signal generator (not shown) configured to generate an ac signal at a pre-determined voltage and an impedance measurement signal processor (not shown) comprising at least an amplifier module and a signal conditioning module (not shown). The impedance measurement signal generator generates the ac signal which is sent to the electrodes 3 and passes through the subject. The electrodes 3 produce impedance data indicative of the impedance of the subject which is passed to the impedance measurement signal processor. The impedance measurement signal processor continuously samples the impedance-indicative data received from the electrodes 3. The amplifier module and the signal conditioning module of the impedance measurement signal processor process the impedance data received from the electrodes 3 by any of amplification, filtering, analogue to digital conversion and signal processing. The impedance signal measurement system 5 thus measures at least one impedance signal of the subject which is passed to the impedance signal processing system 9.

The ECG signal measurement system 7 comprises an ECG measurement signal processor (not shown). When placed on the subject, the electrodes 3 produce ECG data indicative of the ECG of the subject which is passed to the ECG measurement signal processor. The ECG measurement signal processor continuously samples the ECG-indicative data received from the electrodes 3 and then processes the ECG data received from the electrodes 3. The ECG signal measurement system 7 thus measures at least one ECG signal of the subject which is passed to the ECG signal processing system 11.

The impedance signal processing system 9 comprises at least one micro processor which processes the impedance signal to obtain a plurality of impedance signal compression rate estimates and a plurality of impedance signal features. The ECG signal processing system 11 comprises at least one micro processor which processes the ECG signal to obtain a plurality of ECG signal features. The compression rate estimate processing system 13 comprises at least one micro processor which applies a plurality of criteria to the impedance signal features and the ECG signal features and uses compliance with one or more of the criteria to select one of the plurality of impedance signal compression rate estimates as the CPR compression rate. The output unit 15 receives the CPR compression rate and outputs feedback based on the CPR compression rate to a user of the defibrillator.

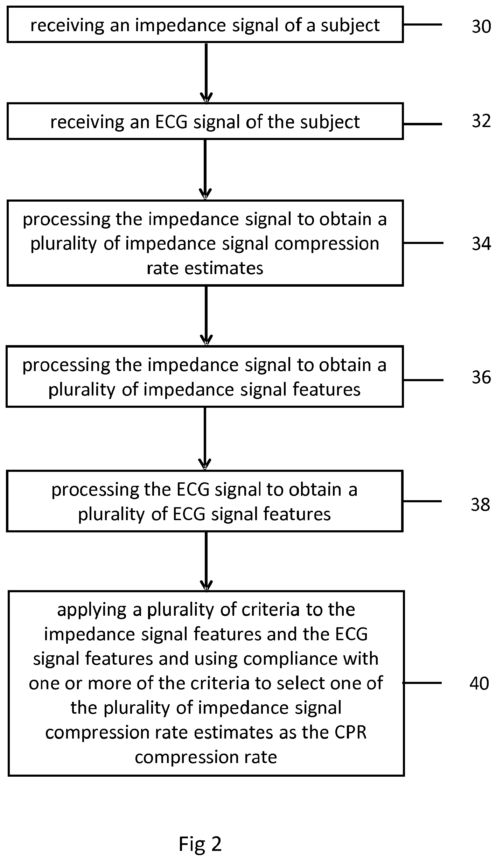

Referring to Figure 2, the method of determining a CPR compression rate carried out by the defibrillator 1 of Figure 1 comprises:
(i) receiving an impedance signal of a subject 30,
(ii) receiving an ECG signal of the subject 32,
(iii) processing the impedance signal to obtain a plurality of impedance signal compression rate estimates 34,
(iv) processing the impedance signal to obtain a plurality of impedance signal features 36,
(v) processing the ECG signal to obtain a plurality of ECG signal features 38, and
(vi) applying a plurality of criteria to the impedance signal features and the ECG signal features and using compliance with one or more of the criteria to select one of the plurality of impedance signal compression rate estimates as the CPR compression rate 40.

Processing the impedance signal to obtain the plurality of impedance signal compression rate estimates 34 comprises using a frequency domain transformation on the impedance signal to obtain an impedance signal frequency spectrum, using a peak detection algorithm to identify a plurality of peaks in the impedance signal frequency spectrum and determining central frequencies of the plurality of peaks as the plurality of impedance signal compression rate estimates.

Processing the impedance signal to obtain the plurality of impedance signal features 36 comprises using a frequency domain transformation on the impedance signal to obtain an impedance signal frequency spectrum, using a peak detection algorithm to identify a plurality of peaks in the impedance signal frequency spectrum and determining central frequencies and amplitudes of the plurality of peaks as the plurality of impedance signal features. Processing the ECG signal to obtain the plurality of ECG signal features 38 comprises using a frequency domain transformation on the ECG signal to obtain an ECG signal frequency spectrum, using a peak detection algorithm to identify a plurality of peaks in the ECG signal frequency spectrum and determining central frequencies and amplitudes of the plurality of peaks as the plurality of ECG signal features.

The transformation uses a FFT algorithm on a six second, advancing window of the impedance signal and a corresponding six second, advancing window of the ECG signal. The peak detection algorithm identifies a plurality of peaks in the impedance signal frequency spectrum and ECG signal frequency spectrum by identifying peaks based on decreasing steepness of slopes of the spectra. In this example, the peak detection algorithm returns two peaks from each frequency spectrum, a primary peak having a highest amplitude at its central frequency and a secondary peak having a next highest amplitude at its central frequency. An amplitude threshold, comprising a pre-determined number of standard deviations above background, is used to determine if the peaks are true peaks. The central frequencies of the primary peak and the secondary peak in the impedance signal frequency spectrum provide the impedance signal compression rate estimates. The central frequencies and amplitudes of the primary peak and the secondary peak in the impedance signal frequency spectrum provide the impedance signal features. The central frequencies and amplitudes of the primary peak and the secondary peak in the ECG signal frequency spectrum provide the ECG signal features.

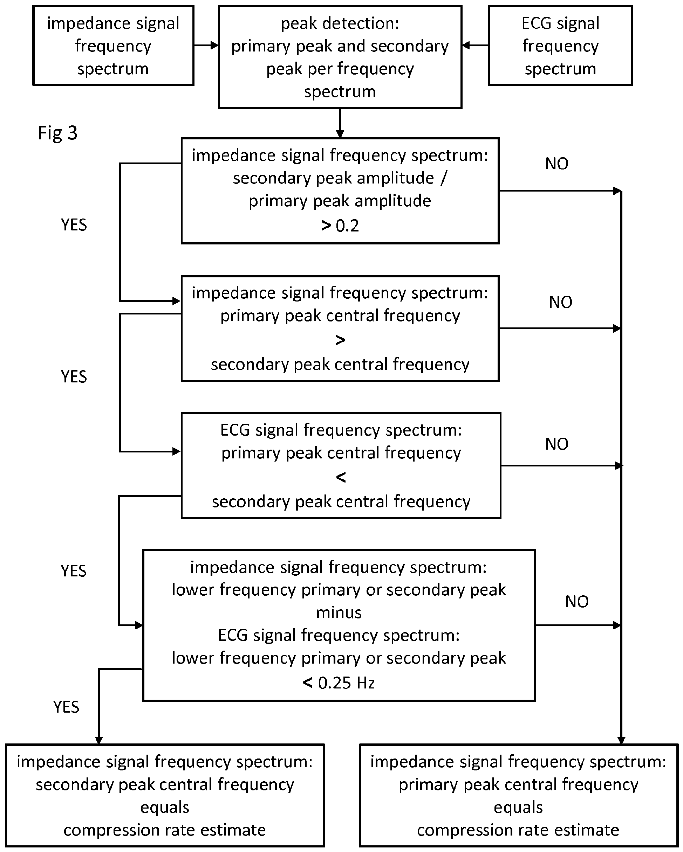

Referring to Figure 3, in this example, the compression rate estimate processing system 13 of the defibrillator 1 applies four criteria to the impedance signal features and the ECG signal features and compliance is used to select one of the impedance signal compression rate estimates as the true CPR compression rate.

The first criterion applied by the compression rate estimate processing system 13 comprises in the impedance signal frequency spectrum a ratio of a secondary peak amplitude and a primary peak amplitude being greater than a pre-determined impedance signal amplitude ratio threshold of 0.2. It will be appreciated that other impedance signal amplitude ratio thresholds may be used.

The second criterion applied by the compression rate estimate processing system 13 comprises in the impedance signal frequency spectrum a central frequency of a primary peak being greater than a central frequency of a secondary peak. The third applied by the compression rate estimate processing system 13 criterion comprises in the ECG signal frequency spectrum a central frequency of a primary peak being less than a central frequency of a secondary peak. The fourth criterion applied by the compression rate estimate processing system 13 comprises a frequency difference between a lower frequency primary or secondary peak in the impedance signal frequency spectrum and a lower frequency primary or secondary peak in the ECG signal frequency spectrum being less than a pre-determined frequency difference threshold of 0.25 Hz. It will be appreciated that other frequency difference thresholds may be used.

The compression rate estimate processing system 13 uses compliance with each of the first to fourth criteria to select an impedance signal compression rate estimate comprising a central frequency of the secondary peak of the impedance signal frequency spectrum as the CPR compression rate, as shown in the figure. Non-compliance with any of the first to fourth criteria is used by the compression rate estimate processing system 13 to select an impedance signal compression rate estimate comprising a central frequency of the primary peak of the impedance signal frequency spectrum as the CPR compression rate, as shown in the figure.

The CPR compression rate thus determined is passed to the output unit 15. This outputs feedback based on the CPR compression rate to the user of the defibrillator 1 comprising an indication that the CPR compression rate is any of satisfactory, too slow, too fast. Outputting the feedback based on the CPR compression rate to the user of the defibrillator provides the user with an opportunity to improve the quality of CPR and gives an indication of the effectiveness of the compressions.

## Claims

1. A defibrillator (1) for determining a rate of cardiopulmonary resuscitation compressions on a subject, comprising
electrodes (3) adapted to be attached to the subject,
an impedance signal measurement system (5) connected to the electrodes (3) and configured to measure at least one impedance signal of the subject,
an electrocardiogram signal measurement system (7) connected to the electrodes (3) and configured to measure at least one electrocardiogram signal of the subject,
an impedance signal processing system (9) connected to the impedance signal measurement system (5) and configured to process the impedance signal of the subject to obtain an impedance signal frequency spectrum and to identify a plurality of peaks in the impedance signal frequency spectrum and determine central frequencies of the plurality of peaks as a plurality of impedance signal compression rate estimates and determine central frequencies and amplitudes of the plurality of peaks as a plurality of impedance signal features,
an electrocardiogram signal processing system (11) connected to the electrocardiogram signal measurement system (7) and configured to process the electrocardiogram signal of the subject to obtain an electrocardiogram signal frequency spectrum and to identify a plurality of peaks in the electrocardiogram signal frequency spectrum and determine central frequencies and amplitudes of the plurality of peaks as a plurality of electrocardiogram signal features,
a compression rate estimate processing system (13) connected to the impedance signal measurement and processing systems (5, 9) and the electrocardiogram signal measurement and processing systems (7, 11) and configured to apply to the impedance signal features and the electrocardiogram signal features a plurality of criteria comprising in the impedance signal frequency spectrum a ratio of a secondary peak amplitude and a primary peak amplitude being greater than a pre-determined impedance signal amplitude ratio threshold,
in the impedance signal frequency spectrum a central frequency of a primary peak being greater than a central frequency of a secondary peak,
in the ECG signal frequency spectrum a central frequency of a primary peak being less than a central frequency of a secondary peak,
a frequency difference between a lower frequency primary or secondary peak in the impedance signal frequency spectrum and a lowerfrequency primary or secondary peak in the ECG signal frequency spectrum being less than a pre-determined frequency difference threshold,
in the ECG signal frequency spectrum a ratio of a secondary peak amplitude and a primary peak amplitude being greater than a pre-determined ECG signal amplitude ratio threshold
and use compliance with each of the criteria to select one of the plurality of impedance signal compression rate estimates comprising a central frequency of a secondary peak of the impedance signal frequency spectrum as the cardiopulmonary resuscitation compression rate, and
an output unit (15) connected to the compression rate estimate processing system and configured to output feedback based on the cardiopulmonary resuscitation compression rate to a user of the defibrillator.

2. A defibrillator (1) according to claim 1 in which the impedance signal processing system (9) processes the impedance signal to obtain the plurality of impedance signal compression rate estimates by using a frequency domain transformation on the impedance signal to obtain the impedance signal frequency spectrum, using a peak detection algorithm to identify the plurality of peaks in the impedance signal frequency spectrum and determine central frequencies of the plurality of peaks as the plurality of impedance signal compression rate estimates.

3. A defibrillator (1) according to claim 1 or claim 2 in which the impedance signal processing system (9) processes the impedance signal to obtain the plurality of impedance signal features by using a frequency domain transformation on the impedance signal to obtain the impedance signal frequency spectrum, using a peak detection algorithm to identify the plurality of peaks in the impedance signal frequency spectrum and determine central frequencies and amplitudes of the plurality of peaks as the plurality of impedance signal features.

4. A defibrillator (1) according to any preceding claim in which the ECG signal processing system (11) processes the ECG signal to obtain the plurality of ECG signal features by using a frequency domain transformation on the ECG signal to obtain the ECG signal frequency spectrum, using a peak detection algorithm to identify the plurality of peaks in the ECG signal frequency spectrum and determine central frequencies and amplitudes of the plurality of peaks as the plurality of ECG signal features.

5. A defibrillator (1) according to any of claims 2 to 4 in which using the frequency domain transformation to obtain the impedance signal frequency spectrum and the ECG signal frequency spectrum comprises using any of a Fast Fourier Transform algorithm on a window of the impedance signal and a corresponding window of the ECG signal, a Goertzel algorithm on a window of the impedance signal and a corresponding window of the ECG signal.

6. A defibrillator (1) according to any of claims 2 to 5 in which using the peak detection algorithm to identify a plurality of peaks in the impedance signal frequency spectrum comprises identifying peaks based on decreasing steepness of slopes of the impedance signal frequency spectrum and using the peak detection algorithm to identify a plurality of peaks in the ECG signal frequency spectrum comprises identifying peaks based on decreasing steepness of slopes of the ECG signal frequency spectrum.

7. A defibrillator (1) according to claim 6 in which the peak detection algorithm returns a primary peak having a highest amplitude at its central frequency, a secondary peak having a next highest amplitude at its central frequency continued up to a specified number of peaks.

8. A defibrillator (1) according to any preceding claim in which the compression rate estimate processing system (13) uses non-compliance with any of the plurality of criteria to select an impedance signal compression rate estimate comprising a central frequency of a primary peak of the impedance signal frequency spectrum as the CPR compression rate.

9. A defibrillator (1) according to any preceding claim which uses substantially all of the measured impedance signal and substantially all of the measured ECG signal to determine a CPR compression rate.

10. A defibrillator (1) according to any preceding claim which uses one or more portions of the measured impedance signal and corresponding one or more portions of the measured ECG signal to determine a CPR compression rate.

11. A defibrillator (1) according to claim 10 in which the or each portion of the measured impedance signal comprises portions which have been pre-analysed for CPR compression rate, which compression rate has not exceeded a threshold used to determine if the rate is a true rate.

12. A method of determining a cardiopulmonary resuscitation (CPR) compression rate, comprising
receiving an impedance signal of a subject,
receiving an electrocardiogram (ECG) signal of the subject,
processing the impedance signal to obtain an impedance signal frequency spectrum and to identify a plurality of peaks in the impedance signal frequency spectrum and determine central frequencies of the plurality of peaks as a plurality of impedance signal compression rate estimates,
processing the impedance signal to obtain the impedance signal frequency spectrum and to identify the plurality of peaks in the impedance signal frequency spectrum and determine central frequencies and amplitudes of the plurality of peaks as a plurality of impedance signal features,
processing the ECG signal to obtain an electrocardiogram signal frequency spectrum and to identify a plurality of peaks in the electrocardiogram signal frequency spectrum and determine central frequencies and amplitudes of the plurality of peaks as a plurality of ECG signal features, and
applying to the impedance signal features and the ECG signal features a plurality of criteria comprising
in the impedance signal frequency spectrum a ratio of a secondary peak amplitude and a primary peak amplitude being greater than a pre-determined impedance signal amplitude ratio threshold,
in the impedance signal frequency spectrum a central frequency of a primary peak being greater than a central frequency of a secondary peak,
in the ECG signal frequency spectrum a central frequency of a primary peak being less than a central frequency of a secondary peak,
a frequency difference between a lower frequency primary or secondary peak in the impedance signal frequency spectrum and a lowerfrequency primary or secondary peak in the ECG signal frequency spectrum being less than a pre-determined frequency difference threshold,
in the ECG signal frequency spectrum a ratio of a secondary peak amplitude and a primary peak amplitude being greater than a pre-determined ECG signal amplitude ratio threshold, and
using compliance with each of the criteria to select one of the plurality of impedance signal compression rate estimates comprising a central frequency of a secondary peak of the impedance signal frequency spectrum as the CPR compression rate.

13. A CPR compression rate determination computer program, tangibly embodied on a computer readable medium, the computer program including instructions for causing a computer to execute the method of determining a rate of CPR compressions according to claim 12.

## Patentansprüche

1. Defibrillator (1) zum Bestimmen einer Rate von Herz-Lungen-Wiederbelebungskompressionen an einem Subjekt, der Folgendes umfasst:
Elektroden (3), ausgelegt zum Anbringen am Subjekt,
ein Impedanzsignal-Messsystem (5), das mit den Elektroden (3) verbunden und zum Messen mindestens eines Impedanzsignals des Subjekts konfiguriert ist,
ein Elektrokardiogrammsignal-Messsystem (7), das mit den Elektroden (3) verbunden und zum Messen mindestens eines Elektrokardiogrammsignals des Subjekts konfiguriert ist,
ein Impedanzsignal-Verarbeitungssystem (9), das mit dem Impedanzsignal-Messsystem (5) verbunden und zum Verarbeiten des Impedanzsignals des Subjekts konfiguriert ist, um ein Impedanzsignal-Frequenzspektrum zu erhalten und mehrere Spitzen im Impedanzsignal-Frequenzspektrum zu identifizieren und Mittenfrequenzen der mehreren Spitzen als mehrere Impedanzsignal-Kompressionsratenschätzungen zu bestimmen und Mittenfrequenzen und Amplituden der mehreren Spitzen als mehrere Impedanzsignalmerkmale zu bestimmen,
ein Elektrokardiogrammsignal-Verarbeitungssystem (11), das mit dem Elektrokardiogrammsignal-Messsystem (7) verbunden und zum Verarbeiten des Elektrokardiogrammsignals des Subjekts konfiguriert ist, um ein Elektrokardiogrammsignal-Frequenzspektrum zu erhalten und mehrere Spitzen in dem Elektrokardiogrammsignal-Frequenzspektrum zu identifizieren und Mittenfrequenzen und Amplituden der mehreren Spitzen als mehrere Elektrokardiogrammsignalmerkmale zu bestimmen,
ein Kompressionsratenschätzverarbeitungssystem (13), das mit den Impedanzsignalmess- und -verarbeitungssystemen (5, 9) und den Elektrokardiogrammsignalmess- und - verarbeitungssystemen (7, 11) verbunden und zum Anwenden mehrerer Kriterien auf die Impedanzsignalmerkmale und die Elektrokardiogrammsignalmerkmale konfiguriert ist, die Folgendes umfassen:
im Impedanzsignal-Frequenzspektrum ist ein Verhältnis einer sekundären Spitzenamplitude und einer primären Spitzenamplitude größer als ein vorbestimmter Impedanzsignalamplitudenverhältnis-Schwellenwert,
im Impedanzsignal-Frequenzspektrum ist eine Mittenfrequenz einer primären Spitze größer als eine Mittenfrequenz einer sekundären Spitze,
im EKG-Signalfrequenzspektrum ist eine Mittenfrequenz einer primären Spitze niedriger als eine Mittenfrequenz einer sekundären Spitze,
eine Frequenzdifferenz zwischen einer primären oder sekundären Spitze der tieferen Frequenz im Impedanzsignal-Frequenzspektrum und einer primären oder sekundären Spitze der tieferen Frequenz im Frequenzspektrum des EKG-Signals ist kleiner als ein vorbestimmter Frequenzdifferenz-Schwellenwert,
im EKG-Signalfrequenzspektrum ist ein Verhältnis einer Amplitude der sekundären Spitze und einer Amplitude der primären Spitze größer als ein vorbestimmter EKG-Signalamplitudenverhältnis-Schwellenwert,
und Nutzen der Erfüllung jedes der Kriterien zum Auswählen von einer der mehreren Impedanzsignal-Kompressionsratenschätzungen mit einer Mittenfrequenz einer sekundären Spitze des Impedanzsignal-Frequenzspektrums als die Herz-Lungen-Wiederbelebungs-Kompressionsrate, und
eine Ausgabeeinheit (15), die mit dem Kompressionsratenschätzungs-Verarbeitungssystem verbunden und zum Ausgeben einer Rückmeldung auf der Basis der Herz-Lungen-Wiederbelebungs-Kompressionsrate an einen Benutzer des Defibrillators konfiguriert ist.

2. Defibrillator (1) nach Anspruch 1, wobei das Impedanzsignal-Verarbeitungssystem (9) das Impedanzsignal zum Erhalten der mehreren Impedanzsignal-Kompressionsratenschätzungen durch Anwenden einer Frequenzbereichstransformation auf das Impedanzsignal zum Erhalten des Impedanzsignal-Frequenzspektrums, Benutzen eines Spitzenerkennungsalgorithmus zum Identifizieren der mehreren Spitzen im Impedanzsignal-Frequenzspektrum und Bestimmen von Mittenfrequenzen der mehreren Spitzen als die mehreren Impedanzsignal-Kompressionsratenschätzungen verarbeitet.

3. Defibrillator (1) nach Anspruch 1 oder Anspruch 2, wobei das Impedanzsignalverarbeitungssystem (9) das Impedanzsignal zum Erhalten der mehreren Impedanzsignalmerkmale durch Anwenden einer Frequenzbereichstransformation auf das Impedanzsignal zum Erhalten des Impedanzsignal-Frequenzspektrums, Benutzen eines Spitzenerkennungsalgorithmus zum Identifizieren der mehreren Spitzen im Impedanzsignal-Frequenzspektrum und Bestimmen von Mittenfrequenzen und Amplituden der mehreren Spitzen als die mehreren Impedanzsignalmerkmale verarbeitet.

4. Defibrillator (1) nach einem vorherigen Anspruch, wobei das EKG-Signalverarbeitungssystem (11) das EKG-Signal zum Erhalten der mehreren EKG-Signalmerkmale durch Anwenden einer Frequenzbereichstransformation auf das EKG-Signal zum Erhalten des EKG-Signalfrequenzspektrums, Benutzen eines Spitzenerfassungsalgorithmus zum Identifizieren der mehreren Spitzen im EKG-Signalfrequenzspektrum und Bestimmen von Mittenfrequenzen und Amplituden der mehreren Spitzen als die mehreren EKG-Signalmerkmale verarbeitet.

5. Defibrillator (1) nach einem der Ansprüche 2 bis 4, wobei die Anwendung der Frequenzbereichstransformation zum Erhalten des Impedanzsignal-Frequenzspektrums und des EKG-Signalfrequenzspektrums die Anwendung eines beliebigen aus einem Fast-Fourier-Transformations-Algorithmus auf ein Fenster des Impedanzsignals und ein entsprechendes Fenster des EKG-Signals, einem Goertzel-Algorithmus auf ein Fenster des Impedanzsignals und ein entsprechendes Fenster des EKG-Signals beinhaltet.

6. Defibrillator (1) nach einem der Ansprüche 2 bis 5, wobei die Anwendung des Spitzenerkennungsalgorithmus zum Identifizieren mehrerer Spitzen im Impedanzsignal-Frequenzspektrum das Identifizieren von Spitzen auf der Basis von abnehmender Steilheit von Flanken des Impedanzsignal-Frequenzspektrums beinhaltet und die Anwendung des Spitzenerkennungsalgorithmus zum Identifizieren mehrerer Spitzen im EKG-Signalfrequenzspektrum das Identifizieren von Spitzen auf der Basis von abnehmender Steilheit von Flanken des EKG-Signalfrequenzspektrums beinhaltet.

7. Defibrillator (1) nach Anspruch 6, wobei der Spitzenerkennungsalgorithmus eine primäre Spitze mit einer höchsten Amplitude auf seiner Mittenfrequenz, eine sekundäre Spitze mit einer nächsthöchsten Amplitude auf seiner Mittenfrequenz, fortfahrend bis zu einer bestimmten Anzahl von Spitzen zurückgibt.

8. Defibrillator (1) nach einem vorherigen Anspruch, wobei das Kompressionsratenschätzverarbeitungssystem (13) Nichterfüllung eines der mehreren Kriterien zum Auswählen einer Impedanzsignal-Kompressionsratenschätzung mit einer Mittenfrequenz einer primären Spitze des Impedanzsignal-Frequenzspektrums als die CPR-Kompressionsrate nutzt.

9. Defibrillator (1) nach einem vorherigen Anspruch, der im Wesentlichen das gesamte gemessene Impedanzsignal und im Wesentlichen das gesamte gemessene EKG-Signal zum Bestimmen einer CPR-Kompressionsrate nutzt.

10. Defibrillator (1) nach einem vorherigen Anspruch, der einen oder mehrere Teile des gemessenen Impedanzsignals und entsprechende ein oder mehrere Teile des gemessenen EKG-Signals zum Bestimmen einer CPR-Kompressionsrate nutzt.

11. Defibrillator (1) nach Anspruch 10, wobei der oder jeder Teil des gemessenen Impedanzsignals Teile umfasst, die für die CPR-Kompressionsrate voranalysiert wurden, wobei die Kompressionsrate einen Schwellenwert nicht überschritten hat, der benutzt wird zum Bestimmen, ob die Rate eine echte Rate ist.

12. Verfahren zum Bestimmen einer Herz-Lungen-Wiederbelebungs-(CPR)-Kompressionsrate, das Folgendes beinhaltet:
Empfangen eines Impedanzsignals eines Subjekts,
Empfangen eines Elektrokardiogramm-(EKG)-Signals des Subjekts,
Verarbeiten des Impedanzsignals zum Erhalten eines Impedanzsignal-Frequenzspektrums und zum Identifizieren mehrerer Spitzen im Impedanzsignal-Frequenzspektrum und Bestimmen von Mittenfrequenzen der mehreren Spitzen als mehrere Impedanzsignal-Kompressionsratenschätzungen,
Verarbeiten des Impedanzsignals zum Erhalten des Impedanzsignal-Frequenzspektrums und zum Identifizieren der mehreren Spitzen im Impedanzsignal-Frequenzspektrum und Bestimmen von Mittenfrequenzen und Amplituden der mehreren Spitzen als mehrere Impedanzsignalmerkmale,
Verarbeiten des EKG-Signals zum Erhalten eines Elektrokardiogrammsignal-Frequenzspektrums und zum Identifizieren mehrerer Spitzen im Elektrokardiogrammsignal-Frequenzspektrum und Bestimmen von Mittenfrequenzen und Amplituden der mehreren Spitzen als mehrere EKG-Signalmerkmale, und
Anwenden mehrerer Kriterien auf die Impedanzsignalmerkmale und die EKG-Signalmerkmale, die Folgendes umfassen:
im Impedanzsignal-Frequenzspektrum ist ein Verhältnis einer sekundären Spitzenamplitude und einer primären Spitzenamplitude größer als ein vorbestimmter Impedanzsignalamplitudenverhältnis-Schwellenwert,
im Impedanzsignal-Frequenzspektrum ist eine Mittenfrequenz einer primären Spitze größer als eine Mittenfrequenz einer sekundären Spitze,
im EKG-Signalfrequenzspektrum ist eine Mittenfrequenz einer primären Spitze niedriger als eine Mittenfrequenz einer sekundären Spitze,
eine Frequenzdifferenz zwischen einer primären oder sekundären Spitze der tieferen Frequenz im Impedanzsignal-Frequenzspektrum und einer primären oder sekundären Spitze der tieferen Frequenz im Frequenzspektrum des EKG-Signals ist kleiner als ein vorbestimmter Frequenzdifferenz-Schwellenwert,
im EKG-Signalfrequenzspektrum ist ein Verhältnis einer Amplitude der sekundären Spitze und einer Amplitude der primären Spitze größer als ein vorbestimmter EKG-Signalamplitudenverhältnis-Schwellenwert,
Nutzen der Erfüllung jedes der Kriterien zum Auswählen von einer der mehreren Impedanzsignal-Kompressionsratenschätzungen mit einer Mittenfrequenz einer sekundären Spitze des Impedanzsignal-Frequenzspektrums als CPR-Kompressionsrate.

13. Computerprogramm zum Bestimmen der Herz-Lungen-Wiederbelebungs-(CPR)-Kompressionsrate, fassbar ausgestaltet auf einem computerlesbaren Medium, wobei das Computerprogramm Befehle enthält, um zu bewirken, dass ein Computer das Verfahren zum Bestimmen einer Rate von CPR-Kompressionen nach Anspruch 12 durchführt.

## Revendications

1. Défibrillateur (1) destiné à déterminer un rythme de compressions de ressuscitation cardio-pulmonaire sur un sujet, comprenant
des électrodes (3) conçues pour être attachées au sujet,
un système de mesure de signal d'impédance (5) connecté aux électrodes (3) et configuré pour mesurer au moins un signal d'impédance du sujet,
un système de mesure de signal d'électrocardiogramme (7) connecté aux électrodes (3) et configuré pour mesurer au moins un signal d'électrocardiogramme du sujet,
un système de traitement de signal d'impédance (9) connecté au système de mesure de signal d'impédance (5) et configuré pour traiter le signal d'impédance du sujet pour obtenir un spectre de fréquences de signal d'impédance et pour identifier une pluralité de pics dans le spectre de fréquences de signal d'impédance et déterminer des fréquences centrales de la pluralité de pics comme une pluralité d'estimations de rythme de compressions de signal d'impédance et déterminer des fréquences centrales et des amplitudes de la pluralité de pics comme une pluralité de caractéristique de signal d'impédance,
un système de traitement de signal d'électrocardiogramme (11) connecté au système de mesure de signal d'électrocardiogramme (7) et configuré pour traiter le signal d'électrocardiogramme du sujet pour obtenir un spectre de fréquences de signal d'électrocardiogramme et pour identifier une pluralité de pics dans le spectre de fréquences de signal d'électrocardiogramme et déterminer des fréquences centrales et des amplitudes de la pluralité de pics comme une pluralité de caractéristiques de signal d'électrocardiogramme,
un système de traitement d'estimations de rythme de compressions (13) connecté aux systèmes de mesure et de traitement de signal d'impédance (5, 9) et aux systèmes de mesure et de traitement de signal d'électrocardiogramme (7, 11) et configuré pour appliquer aux caractéristiques de signal d'impédance et aux caractéristiques de signal d'électrocardiogramme une pluralité de critères comprenant
dans le spectre de fréquences de signal d'impédance un rapport d'une amplitude de pic secondaire et d'une amplitude de pic primaire étant supérieur à un seuil de rapport d'amplitude de signal d'impédance prédéterminé,
dans le spectre de fréquences de signal d'impédance une fréquence centrale d'un pic primaire étant supérieure à une fréquence centrale d'un pic secondaire,
dans le spectre de fréquences de signal d'ECG une fréquence centrale d'un pic primaire étant inférieure à une fréquence centrale d'un pic secondaire,
une différence de fréquence entre un pic primaire ou secondaire de fréquence inférieure dans le spectre de fréquences de signal d'impédance et un pic primaire ou secondaire de fréquence inférieure dans le spectre de fréquences de signal d'ECG étant inférieure à un seuil de différence de fréquence prédéterminé,
dans le spectre de fréquences de signal d'ECG un rapport d'une amplitude de pic secondaire et d'une amplitude de pic primaire étant supérieur à un seuil de rapport d'amplitude de signal d'ECG prédéterminé
et utiliser une conformité avec chacun des critères pour choisir l'une de la pluralité d'estimations de rythme de compressions de signal d'impédance comprenant une fréquence centrale d'un pic secondaire du spectre de fréquences de signal d'impédance comme le rythme de compressions de ressuscitation cardio-pulmonaire, et
une unité de sortie (15) connectée au système de traitement d'estimations de rythme de compressions et configurée pour émettre en sortie un retour d'information basé sur le rythme de compressions de ressuscitation cardio-pulmonaire à destination d'un utilisateur du défibrillateur.

2. Défibrillateur (1) selon la revendication 1 dans lequel le système de traitement de signal d'impédance (9) traite le signal d'impédance pour obtenir la pluralité d'estimations de rythme de compressions de signal d'impédance en utilisant une transformation de domaine fréquentiel sur le signal d'impédance pour obtenir le spectre de fréquences de signal d'impédance, en utilisant un algorithme de détection de pics pour identifier la pluralité de pics dans le spectre de fréquences de signal d'impédance et déterminer les fréquences centrales de la pluralité de pics comme la pluralité d'estimations de rythme de compressions de signal d'impédance.

3. Défibrillateur (1) selon la revendication 1 ou la revendication 2 dans lequel le système de traitement de signal d'impédance (9) traite le signal d'impédance pour obtenir la pluralité de caractéristiques de signal d'impédance en utilisant une transformation de domaine fréquentiel sur le signal d'impédance pour obtenir le spectre de fréquences de signal d'impédance, en utilisant un algorithme de détection de pics dans le spectre de fréquences de signal d'impédance et déterminer les fréquences centrales et les amplitudes de la pluralité de pics comme la pluralité de caractéristiques de signal d'impédance.

4. Défibrillateur (1) selon l'une quelconque des revendications précédentes dans lequel le système de traitement de signal d'ECG (11) traite le signal d'ECG pour obtenir la pluralité de caractéristiques de signal d'ECG en utilisant une transformation de domaine fréquentiel sur le signal d'ECG pour obtenir le spectre de fréquences de signal d'ECG, en utilisant un algorithme de détection de pics pour identifier la pluralité de pics dans le spectre de fréquences de signal d'ECG et déterminer les fréquences centrales et les amplitudes de la pluralité de pics comme la pluralité de caractéristiques de signal d'EGC.

5. Défibrillateur (1) selon l'une quelconque des revendications 2 à 4 dans lequel l'utilisation de la transformation de domaine fréquentiel pour obtenir le spectre de fréquences de signal d'impédance et le spectre de fréquences de signal d'ECG comprend l'utilisation de l'un quelconque d'un algorithme de transformée de Fourier rapide sur une fenêtre du signal d'impédance et une fenêtre correspondante du signal d'ECG, d'un algorithme de Goertzel sur une fenêtre du signal d'impédance et une fenêtre correspondante du signal d'ECG.

6. Défibrillateur (1) selon l'une quelconque des revendications 2 à 5 dans lequel l'utilisation de l'algorithme de détection de pics pour identifier une pluralité de pics dans le spectre de fréquences de signal d'impédance comprend l'identification de pics sur la base de l'inclinaison décroissante de pentes du spectre de fréquences de signal d'impédance et l'utilisation de l'algorithme de détection de pics pour identifier une pluralité de pics dans le spectre de fréquences de signal d'ECG comprend l'identification de pics sur la base de l'inclinaison décroissante de pentes du spectre de fréquences de signal d'ECG.

7. Défibrillateur (1) selon la revendication 6 dans lequel l'algorithme de détection de pics renvoie un pic primaire ayant la plus haute amplitude à sa fréquence centrale, un pic secondaire ayant la deuxième plus haute amplitude à sa fréquence centrale et ainsi de suite jusqu'à un nombre spécifié de pics.

8. Défibrillateur (1) selon l'une quelconque des revendications précédentes dans lequel le système de traitement d'estimations de rythme de compressions (13) utilise la non-conformité avec l'un quelconque de la pluralité de critères pour choisir une estimation de rythme de compressions de signal d'impédance comprenant une fréquence centrale d'un pic primaire du spectre de fréquences de signal d'impédance comme le rythme de compressions de RCP.

9. Défibrillateur (1) selon l'une quelconque des revendications précédentes qui utilise substantiellement l'ensemble du signal d'impédance mesuré et substantiellement l'ensemble du signal d'ECG mesuré pour déterminer un rythme de compressions de RCP.

10. Défibrillateur (1) selon l'une quelconque des revendications précédentes qui utilise une ou plusieurs parties du signal d'impédance mesuré et une ou plusieurs parties correspondantes du signal d'ECG mesuré pour déterminer un rythme de compressions de RCP.

11. Défibrillateur (1) selon la revendication 10 dans lequel la ou chaque partie du signal d'impédance mesuré comprend des parties qui ont été préanalysées pour chaque rythme de compressions de RCP, lequel rythme de compressions n'a pas dépassé un seuil utilisé pour déterminer si le rythme est un vrai rythme.

12. Procédé de détermination d'un rythme de compressions de ressuscitation cardio-pulmonaire (RCP), comprenant
la réception d'un signal d'impédance d'un sujet,
la réception d'un signal d'électrocardiogramme (ECG) du sujet,
le traitement du signal d'impédance pour obtenir un spectre de fréquences de signal d'impédance et pour identifier une pluralité de pics dans le spectre de fréquences de signal d'impédance et déterminer des fréquences centrales de la pluralité de pics comme une pluralité d'estimations de rythme de compressions de signal d'impédance,
le traitement du signal d'impédance pour obtenir le spectre de fréquences de signal d'impédance et pour identifier la pluralité de pics dans le spectre de fréquences de signal d'impédance et déterminer des fréquences centrales et des amplitudes de la pluralité de pics comme une pluralité de caractéristiques de signal d'impédance,
le traitement du signal d'ECG pour obtenir un spectre de fréquences de signal d'électrocardiogramme et pour identifier une pluralité de pics dans le spectre de fréquences de signal d'électrocardiogramme et déterminer des fréquences centrales et des amplitudes de la pluralité de pics comme une pluralité de caractéristiques de signal d'EGC, et
l'application aux caractéristiques de signal d'impédance et aux caractéristiques de signal d'ECG d'une pluralité de critères comprenant
dans le spectre de fréquences de signal d'impédance un rapport d'une amplitude de pic secondaire et d'une amplitude de pic primaire étant supérieur à un seuil de rapport d'amplitude de signal d'impédance prédéterminé,
dans le spectre de fréquences de signal d'impédance une fréquence centrale d'un pic primaire étant supérieure à une fréquence centrale d'un pic secondaire,
dans le spectre de fréquences de signal d'ECG une fréquence centrale d'un pic primaire étant inférieure à une fréquence centrale d'un pic secondaire,
une différence de fréquence entre un pic primaire ou secondaire de fréquence inférieure dans le spectre de fréquences de signal d'impédance et un pic primaire ou secondaire de fréquence inférieure dans le spectre de fréquences de signal d'ECG étant inférieure à un seuil de différence de fréquence prédéterminé,
dans le spectre de fréquences de signal d'ECG un rapport d'une amplitude de pic secondaire et d'une amplitude de pic primaire étant supérieur à un seuil de rapport d'amplitude de signal d'ECG prédéterminé, et
l'utilisation d'une conformité avec chacun des critères pour choisir l'une de la pluralité d'estimations de rythme des compressions de signal d'impédance comprenant une fréquence centrale d'un pic secondaire du spectre de fréquences de signal d'impédance comme le rythme de compressions de RCP.

13. Programme d'ordinateur de détermination d'un rythme de compressions de RCP, mis en œuvre de manière tangible sur un support lisible par ordinateur, le programme d'ordinateur incluant des instructions destinées à amener un ordinateur à exécuter le procédé de détermination d'un rythme de compressions de RCP selon la revendication 12.
